# EUROPEAN PATENT SPECIFICATION

(11) **EP 1 716 831 B1**
(45) Date of publication and mention of the grant of the patent: **07.09.2011**
(21) Application number: 05710309.5
(22) Date of filing: 17.02.2005
(51) Int. Cl.: A61F 13/15, A61F 13/49, A61F 13/494

(54) **DISPOSABLE DIAPER**
EINWEGWINDEL
COUCHE JETABLE

(30) Priority: 17.02.2004 JP 2004040231
(43) Date of publication of application: 02.11.2006
(73) Proprietor: UNI-CHARM CO., LTD., Shikokuchuo, Ehime 799-0111 (JP)
(72) Inventor: SUZUKI, Sachiyo, Technical Center, UNI-CHARM CO. LTD, Kagawa 769-1602 (JP); OTSUBO, Toshifumi, Technical Center, UNI-CHARM CO. LTD, Mitoyo-gun, Kagawa 769-1602 (JP)
(74) Representative: Murgatroyd, Susan Elizabeth
(86) International application number: PCT/JP2005/002459
(87) International publication number: WO 2005/077309

(56) References cited:
- EP-A- 1 358 863
- EP-A- 1 757 256
- WO-A-03/032882
- JP-A- 2000 051 268
- JP-A- 2001 314 440

## Description

### TECHNICAL FIELD OF THE INVENTION

The present invention relates to a disposable diaper and more particularly to the disposable diaper effective to prevent leak of body fluids.

### BACKGROUND ART OF THE INVENTION

There have already been proposed various means adapted to prevent bodily waste such as loose passage or urine from spreading in a transverse direction of the disposable diaper and eventually leaking sideways. For example, the disposable diaper disclosed in U.S. Patent Publication Serial No. 4,695,278 (PATENT DOCUMENT 1) is provided on its inner surface along transversely opposite lateral portions of the diaper with barrier cuffs for prevention of body fluids. The barrier cuffs primarily extend over the crotch covering region and extend further into the front and rear waist covering regions. Each of these barrier cuffs has a proximal edge along which the barrier cuff is bonded to the diaper and a distal edge extending in parallel to the proximal edge and elastically stretchable/contractible in a back-and-forth direction. Rubber threads are contractibly bonded to the distal edge. With this diaper put on the wearer's body, the barrier cuffs are tightly placed around the wearer's thighs to prevent bodily waste from spreading in the transverse direction and thereby to prevent sideways leak of bodily waste from the diaper.

### DISCLOSURE OF THE INVENTION

### PROBLEM TO BE SOLVED BY THE INVENTION

However, after the barrier cuffs have been kept in close contact with the wearer's skin under a contractile force of the rubber threads, these barrier cuffs of the diaper disclosed in PATENT DOCUMENT 1 may leave on the wearer's skin a mark of the rubber threads and/or rash.

In view of the problem as has been described above, it is a principal object of the present invention to provide a disposable diaper provided with leak-barrier means adapted to intercept body fluids from flowing sideways and thereby to prevent body fluids from leaking to the exterior of the diaper improved to eliminate an anxiety that such leak-barrier means might leave on the wearer's skin a mark of rubber thread and/or rash.

### MEASURE TO SOLVE THE PROBLEM AND EFFECT OF THE INVENTION

The object set forth above is achieved, according to the present invention, by an improvement in the disposable diaper having a back-and-forth direction and a transverse direction being orthogonal to each other, the disposable diaper being composed of, in the back-and-forth direction, a crotch covering region, a front waist covering region extending in front of the crotch covering region and a rear waist covering region extending behind the crotch covering region, these covering regions define an inner surface and an outer surface opposed to the inner surface and, on the inner surface, a body fluid absorbent panel comprising a block of body fluid absorbent material and a liquid-pervious sheet wrapping the block extending over the crotch covering region and further to the back-and-forth direction.

The improvement according to the present invention is characterized in that:
said inner surface faces a diaper wearer's skin while said outer surface faces clothes of said wearer;
said body fluid absorbent panel is dimensioned to be narrower than a dimension of said crotch covering region, said panel has a pair of lateral portions opposed to each other in said transverse direction and extending in said back-and-forth direction and a pair of body fluid leak-barrier means are provided in the vicinity of respective said lateral portions so as to extend in said back-and-forth direction;
each of said body fluid leak-barrier means comprises a plurality of strips formed by sheet material having a cantilever stiffness in a range of 10 to 120 as measured in accordance with JIS L 1913, said strips having a thickness direction corresponding to a thickness direction of said sheet material, a width direction corresponding to said back-and-forth direction and a length direction crossing both said thickness direction and said width direction, said strips being arranged side by side in said width direction and overlapping one another in said thickness direction; and
said strips have, as viewed in said length direction, fixed portions bonded to said inner surface or said body fluid absorbent panel and flexible portions lying on the side opposed to said fixed portions.

The disposable diaper according to this embodiment is provided in the vicinity of the transversely opposite lateral portions of the body fluid absorbent panel with the leak-barrier means comprising a plurality cf the strips arranged side by side in the width direction of the respective strips, overlapping one another in the thickness direction of the respective strips and extending substantially from the outer surface toward the inner surface. Bodily waste tending to flow in the transverse direction of the diaper is intercepted by these strips and thus reliably prevented from leaking out of the diaper. None of rubber threads is bonded in stretched state to these strips and therefore the wearer's skin suffers from neither surface imprint nor rash due to the rubber threads even after these strips have been kept in close contact with the wearer's skin. The cantilever stiffness value described herein is the value obtained by averaging cantilever stiffness values measured in accordance with JIS L 1913 using three samples for measurement.

According to one preferred embodiment of the invention, the disposable diaper comprising a pants-shaped or hourglass-shaped chassis having the crotch covering region, the front waist covering region and the rear waist covering region, on one hand, and the body fluid absorbent panel prepared separately of the chassis, on the other hand, and the transversely opposite lateral portions of the body fluid absorbent panel being let free from the inner surface of the chassis in one of the crotch covering region, the front waist covering region and the rear waist covering region, the respective one end portions of the strips being interposed between the inner surface and the lateral portions let free one from another and bonded to the inner surface or the lateral portions while the flexible portions opposite to the one end portions extend outward from the lateral portions in the transverse direction of the chassis.

In the disposable diaper according to this embodiment, the one end portions of the respective strips are interposed between the chassis and the lateral portions of the body fluid absorbent panel and bonded to the chassis or the body fluid absorbent panel, so it is not apprehended that the regions in which the one end portions are bonded to the chassis or the body fluid absorbent panel might irritate the wearer's skin.

According to another preferred embodiment of the invention, each of the strips is bent in the length direction to describe a V-shape opening outward.

The strips are elastically deformable so that the opening angle of the V-shape described by each of the strips may be reduced or enlarged as the disposable diaper according to this embodiment is put en the wearer's body. Consequently, there is substantially no possibility that a gap causing leakage might be left between the skin and the strips.

According to still another preferred embodiment of the invention, the sheet material forming the strips is nonwoven fabric containing therein elastomer fiber or crimped fiber.

In the disposable diaper according to this embodiment, the presence of these fibers enables the strips to be elastically deformable. The strips are reliably kept in contact with the skin as the disposable diaper having such strips is put on the wearer's body, because the strips can be kept in contact with the skin under the elastic deformation. In this way, leak of body waste through the strips and the skin can be reliably prevented.

According to a further preferred embodiment of the invention, a plurality of second strips independent of the body fluid leak-barrier means are arranged side by side in the transverse direction of the disposable diaper between a pair of the body fluid leak-barrier means extending along a pair of the transversely opposite lateral portions to form means adapted to regulate body fluid flow forward or rearward in the back-and-forth direction.

In the disposable diaper according to this embodiment, the regulating means for flow of body fluids can reliably regulate flow of body fluids and therefore it is not apprehended that body fluids might leak beyond the front and rear waist covering regions.

### BRIEF DESCRIPTION OF THE DRAWINGS

[FIG. 1] Fig. 1 is a partially cutaway plan view showing a disposable diaper as a typical embodiment of the invention.
[FIG. 2] Fig. 2 is a sectional view taken along a line II-II in Fig. 1.
[FIG. 3] Fig. 3 is a view similar to Fig. 2, showing the disposable diaper put on the wearer's body.
[FIG. 4] Fig. 4 is a view similar to Fig. 1, showing one preferred embodiment of the invention.
[FIG. 5] Fig. 5 is a sectional view taken along a line V-V in Fig. 4.
[FIG. 6] Fig. 6 is a view similar to Fig. 2, showing another preferred embodiment of the invention
[FIG. 7] Fig. 7 is a view similar to Fig. 2, showing still another preferred embodiment of the invention.
[FIG. 8] Fig. 8 is a sectional view taken along a line VIII-VIII in Fig. 7.
[FIG. 9] Fig. 9 is a perspective view showing a pants-type disposable diaper as further another preferred embodiment of the invention.

### IDENTIFICATION OF REFERENCE NUMERALS USED IN THE DRAWINGS

- 1: diaper
- 2: liquid-pervious sheet (inner sheet)
- 3: liquid-impervious sheet (outer sheet)
- 4: block
- 5: crotch covering region
- 6: front waist covering region
- 7: rear waist covering region
- 21: body fluid leak-barrier means
- 22: strips
- 23: one of opposite end portions (fixed portions)
- 25: the other of opposite end portions (flexible portions)
- 50: chassis
- 51: flow regulating means
- 52: flow regulating means
- 101: diaper
- 201: diaper
- 301: diaper
- 401: diaper
- X: transverse direction
- Y: back-and-forth direction

### DESCRIPTION OF THE BEST MODE FOR WORKING OF THE INVENTION

Details of the disposable diaper according to the present invention will be more fully understood from the description given hereunder in reference with the accompanying drawings.

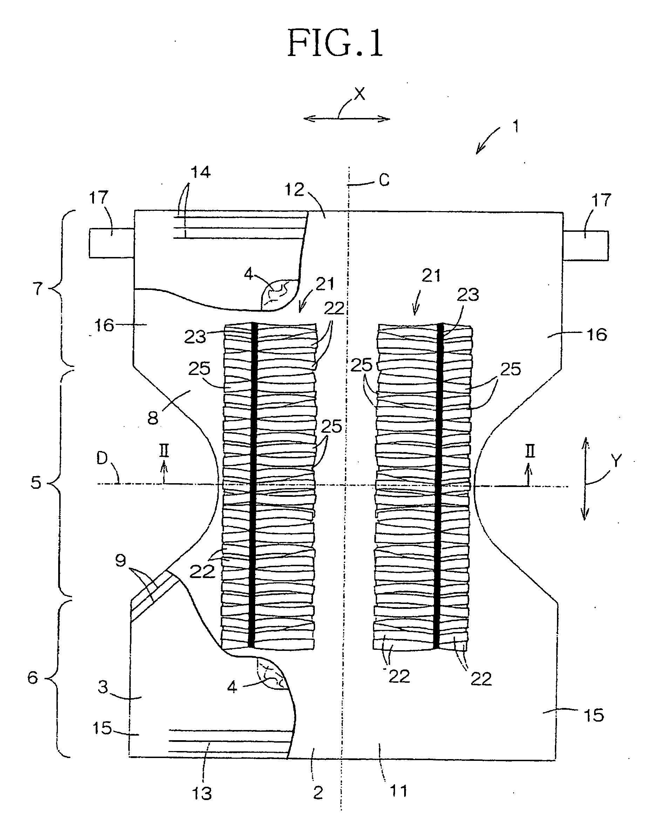

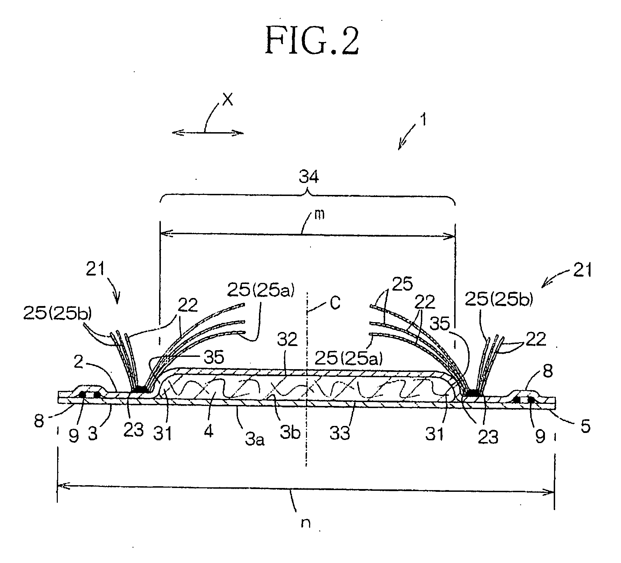

A disposable diaper 1 shown by Fig. 1 in a partially cutaway plan view is of open-type and comprises an inner sheet 2 made of liquid-pervious nonwoven fabric, an outer sheet 3 made of liquid-impervious plastic film and a core 4 formed by a block of liquid-absorbent material and interposed between these sheets 2, 3. The inner and outer sheets 2, 3 extend outward beyond a peripheral edge of the core 4 and respective extensions of these sheets 2, 3 are put flat and bonded together by means of adhesive (not shown). The diaper 1 presents an hourglass-like planar shape extending in a transverse direction X and a back-and-forth direction Y which are orthogonal to each other. In a middle zone of the diaper 1 as viewed in the back-and-forth direction Y, a crotch covering region 5 is defined. Front and rear waist covering regions 6, 7 are respectively defined in front of and behind the crotch covering region 5. The crotch covering region 5 has transversely opposite lateral portions describing circular arcs which are convex toward a center line C bisecting a width of the diaper 1, .e., a dimension of the diaper 1 in the transverse direction X and, along the transversely opposite lateral portions defining side flaps 8 (See Fig. 2), a plurality of rubber threads 9 are interposed between the inner and outer sheets 2, 3 and contractibly bonded to at least one of these sheets 2, 3 by means of hot melt adhesive (not shown). The front and rear waist covering regions 6, 7 respectively have front and rear end portions 11, 12 extending in the transverse direction X and, along these end portions 11, 12, a plurality of rubber threads 13, 14 are interposed between the inner and outer sheets 2, 3 and contractibly bonded to at least one of these sheets 2, 3 by means of hot melt adhesive (not shown). Tape fasteners 17 extend outward from transversely opposite lateral portions 16 of the rear waist covering region 7. To put the diaper 1 on the wearer's body, the tape fasteners 17 may be anchored on the outer sheet 3 in the vicinity of transversely opposite lateral portions 15 of the front waist covering region 6. The diaper 1 further includes a pair of body fluid leak-barrier means 21 laid symmetrically about the center line C. Each of the body fluid leak-barrier means 21 in the embodiment shown by Fig. 1 is formed by an assembly comprising a plurality of strips made of nonwoven fabric which are laid side by side in the back-and-forth direction Y wherein these strips 22 have fixed portions 23 (See Fig. 2) fixed to the inner sheet 2 using a welding technique or appropriate adhesive. The fixed portions 23 welded to the inner sheet 2 and making the strips 22 get out of the initial shapes thereof are indicated in Fig. 1 by band-like thick lines extending in the back-and-forth direction.

Fig. 2 is a sectional view taken along a line II-II corresponding to a center line D bisecting a dimension of the diaper 1 in the back-and-forth dimension. The core 4 extends in the back-and-forth direction over the crotch covering region 5 and further extends into the front and rear waist covering regions 6, 7. The core 4 has transversely opposite lateral portions 31, an inner surface facing the wearer's skin (not shown) and an outer surface 33 facing the wearer's clothes. The lateral portions 31 and the inner surface 32 of the core 4 is covered with the inner sheet 2 and the outer surface of the core 4 is covered with the outer sheet 3 so as to define a body fluid absorbent panel 34. The body fluid absorbent panel 34 has a pair of transversely opposite lateral portions 35 which are opposed to each other in the transverse direction X of the diaper 1 and extending in the back-and-forth direction Y. A dimension m between these lateral portions 35 is smaller than a width n of the crotch covering region 5 and the side flaps 8 adapted to be put in close contact with the wearer's thighs extend outside the respective lateral portions 35. The diaper 1 is further provided in the vicinity of the lateral portions 35 of the body fluid absorbent panel 34 with the leak-barrier means 21 comprising a plurality of the strips 22. Each of these strips 22 has a thickness direction corresponding to a thickness direction of the nonwoven fabric forming this strip 22, a width direction corresponding to the back-and-forth direction Y of the diaper 1 and a length direction which is orthogonal to these thickness direction and width direction. An area of the strip 22 put aside toward one of its longitudinally opposite end portions is bonded to the inner sheet 2 using a welding technique or appropriate adhesive to define the fixed portion 23 and the remaining area extends upward and left free from the inner sheet 2 to define a flexible portion 25. The flexible portion 25 is freely deformable and comes in soft contact with the wearer's skin when the diaper 1 is put on the wearer's body. In the illustrated case, the fixed end portions 23 are welded to the inner sheet 2 and consequently the strips 22 get out of the initial shapes thereof. A plurality of the strips 22 extend in the transverse direction X with the respective flexible portions 25 overlapping one another but appropriately spaced one from another in the back-and-forth direction Y of the diaper 1 and form together a flexible tuft (See Fig. 1 also). These flexible portions 25 forming together the tuft include flexible portions 25a extending obliquely upward as viewed in Fig. 2 from the lateral portions 35 of the body fluid absorbent panel 34 toward the center line C and flexible portions 25b extending upward substantially in a direction orthogonal to the outer sheet 3 illustrated to be horizontal or extending obliquely upward and outward in the transverse direction X. Whether the diaper 1 is for infant or for adult, such strip 22 is preferably dimensioned so that the strip 22 has a width in a range of 3 to 20 mm and the flexible portion 25 has a length in a range of 10 to 80 mm. The length of the flexible portion 25 may be different between the case in which the flexible portion 25 extends toward the center line C and the case in which the flexible portion 25 extends outward with respect to the diaper 1. It should be noted here that, in the case of the flexible portion 25 extending toward the center line C, it length is preferably dimensioned not to extend beyond the center line C as measured with the flexible portion 25 collapsed onto the inner sheet 2 and, in the case of the flexible portion 25 extending outward with respect to the diaper 1, its length is preferably dimensioned not to extend beyond the lateral portion 8 and more preferably dimensioned not to extend beyond the rubber threads 9 surrounding the wearer's thigh as measured with the flexible portion 25 collapsed onto the inner sheet 2.

The nonwoven fabric as stock material for the strip 22 is preferably selected from a group including spun bond nonwoven fabric, spun lace nonwoven fabric and melt blown nonwoven fabric each having a basis weight in a range of 10 to 50 g/m². Such nonwoven fabric preferably contains elastomer fiber or crimped fiber and is elastically deformable. While the nonwoven fabric used for this purpose may be hydrophilic or hydrophobic, the hydrophilic nonwoven fabric allows body fluid to be easily guided through a gap between each pair of the adjacent strips 22 to the body fluid absorbent region 34. However, it is possible without departing from the scope of the invention to form the strips 22 by plastic film or sheet material such as rubber sheet or woven fabric. It is preferable for the strip formed by such material to be elastically deformable. The strip 22 formed by any one selected from the materials as have been described also preferably has a cantilever stiffness value in a range of 10 to 120 as measured in accordance with JIS L 1913 and a plurality of these strips 22 also preferably can form a soft touch tuft.

As will be best seen in Fig. 1, the body fluid leak-barrier means 21 comprising a plurality of the strips 22 extend in parallel to the center line C from the crotch covering region 5 to the front and rear waist covering regions 6, 7. It should be understood here that it is possible for such body fluid leak-barrier means 21 to vary its length in the back-and-forth direction and its position, for example, to lay the body fluid leak-barrier means 21 only in the crotch covering region 5 and the rear waist covering region 7 or only in the rear waist covering region 7 or only in the front waist covering region 6.

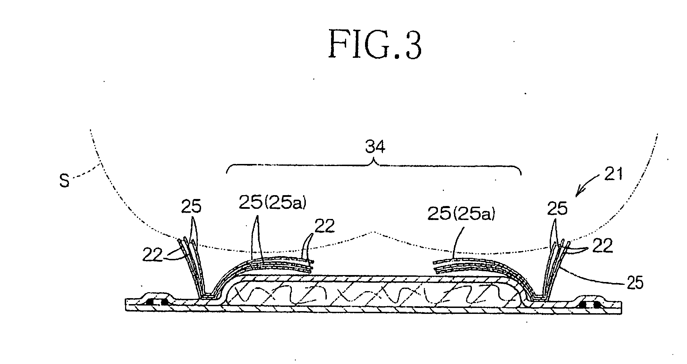

Fig. 3 is a view similar to Fig. 2, in which the diaper 1 is put on the wearer's body and imaginary lines indicate the wearer's skin. The flexible portions 25 of these strips 22 are brought in contact with the skin S and thereby prevent bodily waste (not shown) from leaking beyond the strips 22 out of the diaper 1. mien some of the flexible portions 25 such as those designated by reference numeral 25a in Figs. 2 and 3 lie immediately above the body fluid absorbent panel 34, the strips 22 function to prevent loose passage discharged onto the diaper 1 from spreading on the body fluid absorbent panel 34 in the back-and-forth direction. As a result, there is no anxiety that the wearer's crotch and front waist regions might be contaminated with loose passage.

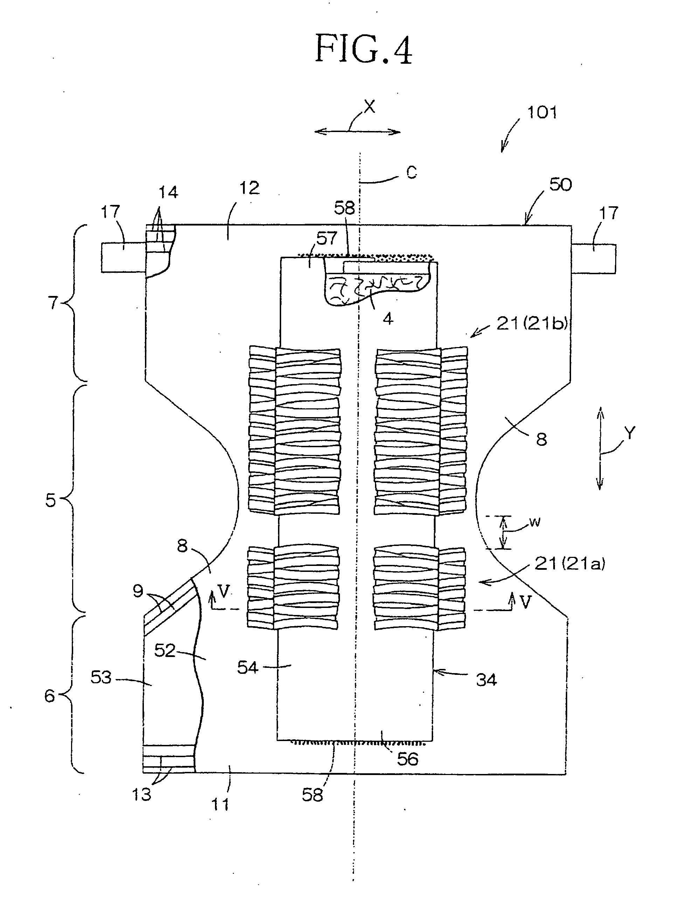

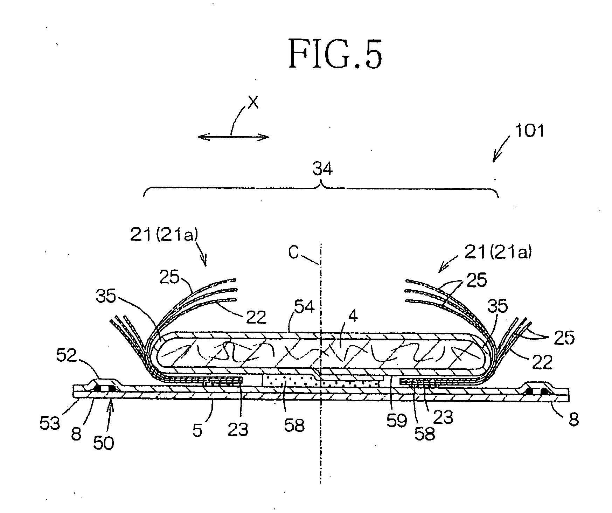

Fig. 4 is a view similar to Fig. 1, showing one preferred embodiment of the invention and Fig. 5 is a sectional view taken along a line V-V in Fig. 4. A diaper 101 according to this embodiment presents an hourglass-like planar shape and comprises a chassis 50 having the crotch covering region 5, the front waist covering region 6 and a rear waist covering region 7, and the body fluid absorbent panel 34 prepared separately of the chassis 50 and has a rectangular shape which is relatively long in the back-and-forth direction Y.

The chassis 50 has an inner surface defined by liquid-impervious plastic film 52 and an outer surface defined by nonwoven fabric 53. Transversely opposite lateral portions of a crotch covering region 5 describe circular arcs which are convex toward a center line C bisecting a dimension of the diaper 101 in the transverse direction to define the side flaps 8 destined to surround the wearer's thighs. A plurality of rubber threads 9 are interposed between the film 52 and the nonwoven fabric 53 along these side flaps 8 and contractibly bonded to at least one of these film 52 and nonwoven fabric 53. Along the front and rear end portions 11, 12 extending in the transverse direction in the front and rear waist covering regions 6, 7, respectively, a plurality of rubber threads 13, 14 extending in the transverse direction X along these front and rear end portions 11, 12 are interposed between the film 52 and the nonwoven fabric 53 and contractibly bonded to at least one of these film 52 and nonwoven fabric 53.

The body fluid absorbent panel 34 comprises the core 4 in the form of a block made from body fluid absorbent material such as fluff pulp and super-absorbent polymer particles and a liquid-pervious sheet 54 such as nonwoven fabric or perforated plastic film with which the core 4 is wrapped. Front and rear end portions 56, 57 of the body fluid absorbent panel 34 are formed by upper and lower layers of the core wrapping liquid-pervious sheet 54 put flat and bonded together. These front and rear end portions 56, 57 are bonded to the film 52 by means of adhesive 58. While the body fluid absorbent panel 34 is bonded to the film 52 by means of adhesive 58 along a transversely middle zone of its lower surface facing the film 52 (See Fig. 5), the panel 34 is not bonded to the film 52 in the vicinity of the transversely opposite lateral portions 35 of the panel 34, i.e., left free from the film 52.

In the case of this diaper 101, the leak-barrier means 21 comprises first leak-barrier means 21a extending from the crotch covering region 5 to the front waist covering region 6 of the chassis 50 and second leak-barrier means 21b extending from the crotch covering region 5 to the rear waist covering region 7 wherein the first and second leak-barrier means 21a, 21b are spaced from each other in the back-and-forth direction Y by a dimension W. The first leak-barrier means 21a are formed so as to lie in the vicinity of urethral and the second leak-barrier means 21b are formed so as to lie in the vicinity of anus. Construction of the first leak-barrier means 21a is substantially similar to construction of the second leak-barrier means 21b. Fig. 5 shows a cross-sectional shape of the first leak-barrier means 21a. In the case of the first leak-barrier means 21a, the fixed portions 23 of the respective strips 22 lie under the transversely opposite lateral portions 35 of the body fluid absorbent panel 34 and fixed to the plastic film 52 using a welding technique or appropriate adhesive. In this specific embodiment illustrated by Figs. 4 and 5, the fixed portions 23 are bonded to the plastic film 52 by means of adhesive 58. The flexible portions 25 of the respective strips 22 extend from the vicinity of the transversely opposite lateral portions 35 of the body fluid absorbent panel 34 in the transverse direction X of the chassis 50. Some of these strips 22 have the flexible portions 25 thereof curve along the transversely opposite lateral portions 35 toward the center line C and the other have the flexible portions 25 thereof extend toward the side flaps of the chassis 50. The flexible portions 25 may be curved as in the illustrated embodiment by thermoforming the strips 22 or by bonding the flexible portions 25 to the liquid-pervious sheet 54 in the vicinity of the transversely opposite lateral portions 35.

With the diaper 101 arranged as illustrated by Figs. 4 and 5, it is not apprehended that the fixed portions 23 of the respective strips 22 might come contact with the wearer's skin and irritate the wearer's skin. The feature that the first and second leak-barrier means 21a, 21b are spaced from each other in the back-and-forth direction in the crotch covering region 5 by the dimension W allows bulkiness of the crotch covering region 5 due to the presence of the leak-barrier means to be alleviated. The dimension W is preferably in a range of 10 to 100 mm.

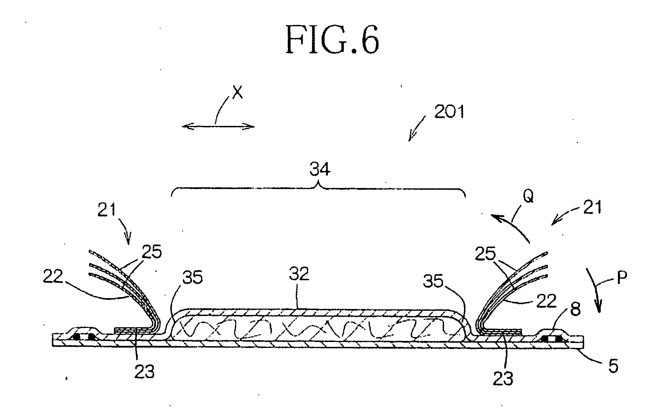

Fig. 6 is a view similar to Fig. 2, showing another preferred embodiment of the invention. In the case of a diaper 201 according to this embodiment, the respective strips 22 constituting the leak-barrier means 21 are postured in V-shape opening outward. The fixed portions 23 are bonded to the side flaps 8 of the diaper 201 by means of adhesive (not shown) and the flexible portions 25 extend outward from the vicinity of the transversely opposite lateral portions 35 of the body fluid absorbent panel 34. The strips 22 come in contact with the wearer's skin (not shown) and thereby pressed down in a direction indicated by an arrow P as the diaper 201 is put on the wearer's body. Thereupon, a counteractive force of restoration is exerted on the strips 22 in a direction Q opposite to the direction P and thereby the strips 22 are brought in close contact with the skin substantially with no gap between the strips 22 and the skin. The strips 22 functioning in this manner are preferably made of elastically deformable nonwoven fabric containing elastomer fiber or crimped fiber.

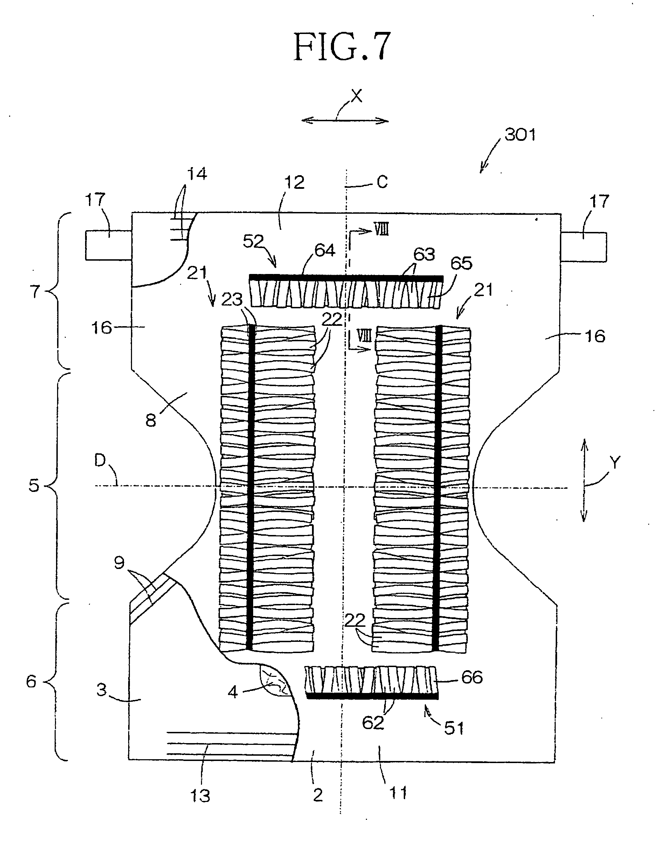

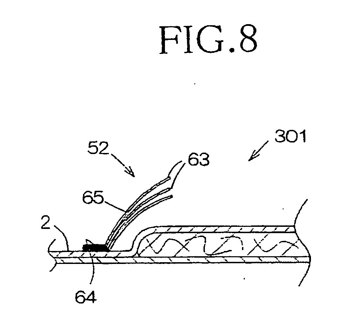

Fig. 7 is a view similar to Fig. 2, showing still another preferred embodiment of the invention and Fig. 8 is a sectional view taken along a line VIII-VIII in Fig. 7. A diaper 301 according to this embodiment includes, in addition to a pair of leak-barrier means 21 corresponding to the above described first leak-barrier means 21 each comprising a plurality of the first strips made of nonwoven fabric similarly to the case illustrated by Fig. 1, front and rear flow regulating means 51, 52 in the front and rear waist covering regions 6, 7, respectively. These front and rear flow regulating means 51, 52 extend in the transverse direction X between the pair of leak-barrier means 21. These regulating means 51, 52 respectively comprise a plurality of second and third strips 62, 63 prepared separately of the first strips 22 and made of sheet material such as nonwoven fabric, plastic film or rubber sheet, more preferably made of elastically deformable nonwoven fabric. These second and third strips 62, 63 are arranged side by side in the transverse direction X and extend in the back-and-forth direction with flexible portions overlapping one another. As will be apparent from Fig. 8, the third strips 63 constituting the rear flow regulating means 52 have lost the initial shapes as the strips 63 after fixed portions 64 thereof defined by proximal end portions have been welded to the inner sheet 2. The flexible portions 65 defined by the end portions opposite to the fixed portions 64 extend above the inner sheet 2. In the preferred rear flow regulating means 52, the flexible portions 65 in majority of the third strips 63 extend upward obliquely or so as to describe curves toward the center line D (See Fig. 7) . Construction of the front flow regulating means 51 is substantially same as the construction of the rear flow regulating means 52 and, in the preferred front flow regulating means 51 also, the flexible portions 66 in majority of the second strips 62 extend upward obliquely or so as to describe curves toward the center line D.

The front and rear flow regulating means 51, 52 in the diaper 301 constructed in the manner as has been described above in reference with Figs. 7 and 8 function to regulate a range in which bodily waste (not shown) can flow on the inner sheet 2 in the back-and-forth direction. The front flow regulating means 51 is adapted to prevent body fluid from flowing forward until body fluid might leak beyond the front end portion 11 of the front waist covering region 6. The rear flow regulating means 52 is adapted to prevent body fluid from flowing rearward until body fluid might leak beyond the rear end portion 12 of the rear waist covering region 7. It should noted here that, if it is unnecessary to prevent body fluid from leaking beyond the front end portion 11 or rear end portions 12, it is possible to eliminate the front flow regulating means 51 or the rear flow regulating means 52 from the diaper 301. The second and third strips 62, 63 may be made of good touch flexible nonwoven fabric as the first strips 22 are the case. With such strips 62, 63, there is no anxiety that visible marks or rash might be left on the wearer's skin even after these strips 62, 63 have been kept in close contact with the wearer's skin.

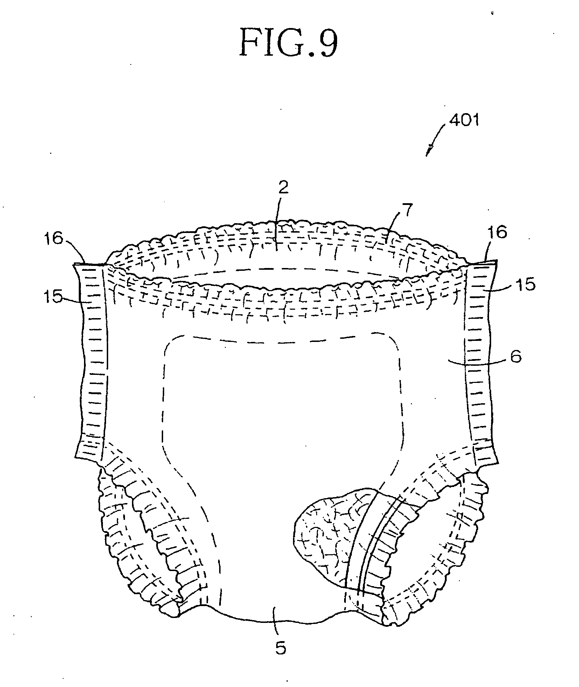

Fig. 9 is a perspective view showing a pants-type disposable diaper 401 as further another preferred embodiment of the invention. Such pants-type diaper 401 may be obtained by folding the open-type diaper shown by Fig. 1 or 4 in the plan view along the center line D with the inner sheet 2 inside to put flat the transversely opposite lateral portions 15, 16 of the front and rear waist covering regions 6, 7 together, and bonding these lateral portions 15, 16 to each other using adhesive or appropriate welding technique. No tape fastener is necessary for the diaper 401, so the tape fasteners 17 may be previously removed from the diaper when it is desired to obtain the open-type diaper as illustrated by Fig. 1 or 4.

### INDUSTRIAL APPLICABILITY

The present invention is applicable to produce a disposable diaper provided with leak-barrier means free from the anxiety of irritating the wearer's skin.

## Claims

1. A disposable diaper having a back-and-forth direction (Y) and a transverse direction (X) being orthogonal to each other, said disposable diaper being composed of, in said back-and-forth direction (Y), a crotch covering region (5), a front waist covering region (6) extending in front of said crotch covering region (5) and a rear waist covering region (7) extending behind said crotch covering region (5), these covering regions define an inner surface (2) and an outer surface (3) opposed to said inner surface (2) and, on said inner surface (2), a body fluid absorbent panel (34) comprising a block (4) of body fluid absorbent material and a liquid-pervious sheet (2) wrapping said block (4) extending over said crotch covering region (5) and further in said back-and-forth direction (Y), said disposable diaper being **characterized in that**:
said inner surface (2) faces a diaper wearer's skin while said outer surface (3) faces clothes of said wearer;
said body fluid absorbent panel (34) is dimensioned to be narrower than a dimension of said crotch covering region (5), said panel (34) has a pair of lateral portions (35) opposed to each other in said transverse direction (X) and extending in said back-and-forth direction (Y) and a pair of body fluid leak-barrier means (21) are provided in the vicinity of respective said lateral portions (35) so as to extend in said back-and-forth direction (Y);
each of said body fluid leak-barrier means (21) comprises a plurality of strips (22) formed by sheet material having a cantilever stiffness in a range of 10 to 120 as measured in accordance with JIS L 1913, said strips (22) having a thickness direction corresponding to a thickness direction of said sheet material, a width direction corresponding to said back-and-forth direction (Y) and a length direction crossing both said thickness direction and said width direction, said strips (22) being arranged side by side in said width direction and overlapping one another in said thickness direction; and
said strips (22) have, as viewed in said length direction, fixed portions (23) bonded to said inner surface (2) or said body fluid absorbent panel (34) and flexible portions (25) lying on the side opposed to said fixed portions (23).

2. The disposable diaper as set forth by Claim 1, wherein said disposable diaper comprising a pants-shaped or hourglass-shaped chassis having said crotch covering region (5), said front waist covering region (6) and said rear waist covering region (7), on one hand, and said body fluid absorbent panel (34) prepared separately of said chassis, on the other hand, said transversely opposite lateral portions (35) of said body fluid absorbent panel (34) being let free from said inner surface (2) of said chassis in one of said crotch covering region (5), said front waist covering region (6) and said rear waist covering region (7), respective said one end portions (23) of said strips (22) being interposed between said inner surface (2) and said lateral portions (35) let free one from another and bonded to said inner surface (2) or said lateral portions (35) while said flexible portions (25) opposite to said one end portions (23) extend outward from said lateral portions (35) in said transverse direction (X) of said chassis.

3. The disposable diaper as set forth by Claim 1 or 2, wherein each of said strips (22) is bent in said length direction to describe a V-shape opening outward.

4. The disposable diaper as set forth by any one of Claims 1 through 3, wherein said sheet material forming said strips (22) is nonwoven fabric containing therein elastomer fiber or crimped fiber.

5. The disposable diaper as set forth by any one of Claims 1 through 4, wherein a plurality of second strips (62, 63) independent of said body fluid leak-barrier means (21) are arranged side by side in said transverse direction (X) of said disposable diaper between a pair of said body fluid leak-barrier means (21) extending along a pair of said transversely opposite lateral portions (35) to form means (51,52) adapted to regulate body fluid flow forward or rearward in said back-and-forth direction (Y).

## Patentansprüche

1. Einwegwindel mit einer Rück-Vor-Richtung (Y) und einer Querrichtung (X), die zueinander senkrecht sind, wobei die Einwegwindel zusammengesetzt ist aus - in der Rück-Vor-Richtung (Y) - einem Schrittabdeckungsbereich (5), einem vorderen Hüftabdeckungsbereich (6), der sich vor dem Schrittabdeckungsbereich (5) erstreckt, und einem hinteren Hüftabdeckungsbereich (7), der sich hinter dem Schrittabdeckungsbereich (5) erstreckt, wobei diese Abdeckungsbereiche eine Innenoberfläche (2) und eine der Innenoberfläche (2) entgegengesetzte Außenoberfläche (3) definieren, sowie auf der Innenoberfläche (2) einem körperflüssigkeitsabsorbierenden Kissen (34), das einen Block (4) aus körperflüssigkeitsabsorbierendem Material und ein flüssigkeitsdurchlässiges Flachbahnmaterial (2) umfasst, das den Block (4) einwickelt, der sich über den Schrittabdeckungsbereich (5) und weiter in der Rück-Vor-Richtung (Y) erstreckt, wobei die Einwegwindel **dadurch gekennzeichnet ist, dass**:
die Innenoberfläche (2) der Haut eines Trägers der Windel zugewandt ist, während die Außenoberfläche (3) der Kleidung des Trägers zugewandet ist;
das körperflüssigkeitsabsorbierende Kissen (34) so abgemessen ist, dass es schmaler als eine Abmessung des Schrittabdeckungsbereichs (5) ist, wobei das Kissen (34) ein Paar Seitenteile (35) aufweist, die sich in der Querrichtung (X) gegenüber liegen und sich in der Rück-Vor-Richtung (Y) erstrecken, und ein Paar Körperflüssigkeits-Austritts-Sperr-Mittel (21) in der Nähe der jeweiligen Seitenteile (35) vorgesehen sind, um sich so in der Rück-Vor-Richtung (Y) zu erstrecken;
wobei jedes der Körperflüssigkeits-Austritts-Sperr-Mittel (21) eine Vielzahl von Streifen (22) umfasst, die aus einem Flachbahnmaterial ausgebildet sind, das gemessen nach der Norm JIS L 1913 eine Kragarmsteifigkeit im Bereich von 10 bis 120 hat, wobei die Streifen (22) eine Dickenrichtung haben, die einer Dickenrichtung des Flachbahnmaterials entspricht, eine Breitenrichtung haben, die der Rück-Vor-Richtung (Y) entspricht, und eine Längenrichtung haben, die sowohl die Dickenrichtung als auch die Breitenrichtung kreuzt, wobei die Streifen (22) in der Breitenrichtung nebeneinander angeordnet sind und sich in der Dickenrichtung miteinander überlagern; und
die Streifen (22) in der Längenrichtung gesehen befestigte Teile (23) haben, die mit der Innenoberfläche (2) des körperflüssigkeitsabsorbierenden Kissens (34) verbunden sind, und flexible Teile (25) haben, die auf der den befestigten Teilen (23) gegenüber liegenden Seite liegen.

2. Einwegwindel nach Anspruch 1, wobei einerseits die Einwegwindel eine höschenförmige oder sanduhrförmige Basiswindel umfasst, die den Schrittabdeckungsbereich (5), den vorderen Hüftabdeckungsbereich (6) und den hinteren Hüftabdeckungsbereich (7) aufweist, und andererseits das körperflüssigkeitsabsorbierende Kissen (34) getrennt von der Basiswindel ausgebildet wird, wobei die sich in Querrichtung gegenüber liegenden Seitenteile (35) des körperflüssigkeitsabsorbierenden Kissens (34) im Schrittabdeckungsbereich (5), im vorderen Hüftabdeckungsbereich (6) oder im hinteren Hüftabdeckungsbereich (7) von der Innenoberfläche (2) der Basiswindel lose gelassen sind, die entsprechenden einen Endteile (23) der Streifen (22), die zwischen der Innenoberfläche (2) und den Seitenteilen (35) angeordnet, voneinander lose gelassen sind und mit der Innenoberfläche (2) der Seitenteile (35) verbunden sind, während die flexiblen Teile (25), die den einen Endteilen (23) gegenüber liegen, sich von den Seitenteilen (35) in der Querrichtung (X) der Basiswindel nach außen erstrecken.

3. Einwegwindel nach Anspruch 1 oder 2, wobei jeder der Streifen (22) in der Längenrichtung gebogen ist, um nach außen eine V-förmige Öffnung zu beschreiben.

4. Einwegwindel nach einem der Ansprüche 1 bis 3, wobei das die Streifen (22) bildende Flachbahnmaterial ein Vliesstoff ist, in dem Elastomerfasern oder Kräuselfasern enthalten sind.

5. Einwegwindel nach einem der Ansprüche 1 bis 4, wobei eine Vielzahl von zweiten Streifen (62, 63) unabhängig von den Körperflüssigkeits-Austritts-Sperr-Mitteln (21) in der Querrichtung (X) der Einwegwindel nebeneinander zwischen einem Paar der Körperftüssigkeits-Austritts-Sperr-Mittel (21) angeordnet sind, die sich entlang einem Paar der sich quer gegenüber liegenden Seitenteilen (35) erstrecken, um Mittel (51, 52) zu bilden, die dazu ausgelegt sind, einen Körperflüssigkeitsfluss in der Rück-Vor-Richtung (Y) vorwärts oder rückwärts zu regulieren.

## Revendications

1. Couche jetable ayant une direction longitudinale (Y) et une direction transversale (X) qui sont orthogonales entre elles, ladite couche jetable étant composée, dans ladite direction longitudinale (Y), d'une région de recouvrement d'entrejambe (5), d'une région de recouvrement de taille avant (6) s'étendant devant la région de recouvrement d'entrejambe (5) et d'une région de recouvrement de taille arrière (7) s'étendant derrière ladite région de recouvrement d'entrejambe (5), ces régions de recouvrement définissent une surface interne (2) et une surface externe (3) opposée à ladite surface interne (2) et, sur ladite surface interne (2), un panneau d'absorption de fluide corporel (34) comprenant un bloc (4) de matériau d'absorption de fluide corporel et une feuille perméable au liquide (2) enveloppant ledit bloc (4) s'étendant sur ladite région de recouvrement d'entrejambe (5) et en outre dans ladite direction longitudinale (Y), ladite couche jetable étant **caractérisée en ce que** :
ladite surface interne (2) est tournée vers la peau de l'utilisateur de la couche alors que ladite surface externe (3) est tournée vers le vêtement dudit utilisateur ;
ledit panneau d'absorption de fluide corporel (34) est dimensionné pour être plus étroit qu'une dimension de ladite région de recouvrement d'entrejambe (5), ledit panneau (34) a une paire de parties latérales (35) opposées entre elles dans ladite direction transversale (X) et s'étendant dans ladite direction longitudinale (Y) et une paire de moyens de barrière aux fuites de fluide corporel (21) sont prévus à proximité desdites parties latérales (35) respectives de manière à s'étendre dans ladite direction longitudinale (Y) ;
chacun desdits moyens de barrière aux fuites de fluide corporel (21) comprenant une pluralité de bandes (22) formées par un matériau en feuille ayant une rigidité en porte-à-faux de l'ordre de 10 à 120, mesurée selon la norme JIS L 1913, lesdites bandes (22) ayant une direction d'épaisseur correspondant à une direction d'épaisseur dudit matériau en feuille, une direction de largeur correspondant à ladite direction longitudinale (Y) et une direction de longueur traversant à la fois ladite direction d'épaisseur et ladite direction de largeur, lesdites bandes (22) étant agencées côte à côte dans ladite direction de largeur et se chevauchant dans ladite direction d'épaisseur ; et
lesdites bandes (22) ont, lorsqu'elles sont observées dans ladite direction de longueur, des parties fixes (23) reliées à ladite surface interne (2) ou audit panneau d'absorption de fluide corporel (34) et des parties flexibles (25) se trouvant sur le côté opposé auxdites parties fixes (23).

2. Couche jetable selon la revendication 1, dans laquelle ladite couche jetable comprend une forme de culotte ou un châssis en forme de sablier ayant ladite région de recouvrement d'entrejambe (5), ladite région de recouvrement de taille avant (6) et ladite région de recouvrement de taille arrière (7) d'une part, et ledit panneau d'absorption de fluide corporel (34) préparé séparément dudit châssis, d'autre part, lesdites parties latérales transversalement opposées (35) dudit panneau d'absorption de fluide corporel (34) étant libres de ladite surface interne (2) dudit châssis dans l'une parmi ladite région de recouvrement d'entrejambe (5), ladite région de recouvrement de taille avant (6) et ladite région de recouvrement de taille arrière (7), respectivement, ladite partie d'extrémité (23) desdites bandes (22) étant intercalée entre ladite surface interne (2) et lesdites parties latérales (35) libres les unes par rapport aux autres et reliées à ladite surface interne (2) ou lesdites parties latérales (35) alors que lesdites parties flexibles (25) opposées auxdites parties d'extrémité (23) s'étendent vers l'extérieur à partir desdites parties latérales (35) dans ladite direction transversale (X) dudit châssis.

3. Couche jetable selon la revendication 1 ou 2, dans laquelle chacune desdites bandes (22) est pliée dans ladite direction de la longueur pour décrire une ouverture en forme de V vers l'extérieur.

4. Couche jetable selon l'une quelconque des revendications 1 à 3, dans laquelle ledit matériau en feuille formant lesdites bandes (22) est un tissu non tissé contenant à l'intérieur de ce dernier des fibres élastomères ou des fibres crêpées.

5. Couche jetable selon l'une quelconque des revendications 1 à 4, dans laquelle une pluralité de deuxièmes bandes (62, 63) indépendantes desdits moyens de barrière aux fuites de fluide corporel (21) sont agencées côte à côte dans ladite direction transversale (X) de ladite couche jetable entre une paire desdits moyens de barrière aux fuites de fluide corporel (21) s'étendant le long d'une paire de parties latérales transversalement opposées (35) afin de former des moyens (51, 52) adaptés pour réguler le flux de fluide corporel vers l'avant ou vers l'arrière dans ladite direction longitudinale (Y).
